# EUROPÄISCHE PATENTSCHRIFT

(11) **EP 2 453 929 B1**
(45) Veröffentlichungstag und Bekanntmachung des Hinweises auf die Patenterteilung: **11.10.2017**
(21) Anmeldenummer: 10718967.2
(22) Anmeldetag: 06.05.2010
(51) Int. Cl.: A61L 9/12

(54) **DUFTABGABESYSTEM MIT AKTIVIERUNGS- UND/ODER VERBRAUCHSANZEIGE**
FRAGRANCE DISPENSING SYSTEM WITH ACTIVATION INDICATOR AND/OR CONSUMPTION INDICATOR
SYSTÈME DE DISTRIBUTION DE PARFUM DOTÉ D'UN AFFICHAGE D'ACTIVATION ET/OU DE CONSOMMATION

(30) Priorität: 08.10.2009 DE 102009045482
(43) Veröffentlichungstag der Anmeldung: 23.05.2012
(73) Patentinhaber: Henkel AG & Co. KGaA, 40589 Düsseldorf (DE)
(72) Erfinder: SUNDER, Matthias, 40593 Düsseldorf (DE); MEIER, Frank, 40589 Düsseldorf (DE); FREEBORN, Matthew, 85260 Scottsdale (DE)
(86) Internationale Anmeldenummer: PCT/EP2010/056137
(87) Internationale Veröffentlichungsnummer: WO 2011/042222

(56) Entgegenhaltungen:
- EP-A2- 0 309 173
- EP-A2- 0 462 605
- WO-A1-2004/006968
- US-A1- 2008 251 599

## Beschreibung

Die Erfindung betrifft ein diffusionsgetriebenes Duftabgabesystem mit einer Aktivierungs- und/oder Verbrauchsanzeige.

### Stand der Technik

Diffusionsgetriebene Duftabgabesysteme sind hinlänglich aus dem Stand der Technik bekannt. Insbesondere sind Systeme basierend auf einem Docht und einem mit flüssiger, duftstoffhaltiger Zubereitung befülltem Behälter bekannt, bei denen der Docht in den Behälter eingeführt und die Zubereitung aufgrund der Kapillarkräfte des Dochtes entgegen der Schwerkraftrichtung zu den Oberflächen des Dochtes geführt wird, von wo aus der so transportierte Duftstoff an die Umgebung, üblicherweise über Verdampfung, abgegeben wird (zum Beispiel WO 2004/006968 A1 und US 2008/0251599 A1). Nachteilig an derartigen Duftabgabesystemen ist es, dass vom Benutzer nicht erkannt werden kann, ob das System nach seiner erstmaligen Aktivierung korrekt funktioniert - also bestimmungsgemäß an die Umgebung abgibt - und/oder wann sich das System verbraucht hat.

### Aufgabe der Erfindung

Aufgabe der Erfindung ist es daher, ein Duftabgabesystem bereit zu stellen, dass auf einfache Weise einem Benutzer anzeigt, dass das Duftabgabesystem korrekt aktiviert wurde und/oder sich das Duftabgabesystem verbraucht hat.

Diese Aufgabe wird durch ein Duftabgabesystem mit den Merkmalen des Anspruchs 1 gelöst.

Das erfindungsgemäße Duftabgabesystem umfasst einen Docht und mindestens einen Diffusor, wobei die mit Umgebungsluft in Kontakt stehende Oberfläche des Diffusors größer ist als die mit der Umgebungsluft in Kontakt stehende Oberfläche des Dochtes. Somit bewirkt der Diffusor eine Vergrößerung der Oberfläche, über die Duftstoff an die Umgebung abgegeben wird.

Das erfindungsgemäße Duftabgabesystem umfasst des Weiteren einen Behälter zur Aufnahme des Dochts, sowie eine Duftstoffzubereitung, die in dem Behälter bevorratet ist, wobei die Duftstoffzubereitung in dem im Behälter aufgenommenen Zustand des Dochtes mittels Kapillarwirkung entgegen der Schwerkraftrichtung durch den Docht zum Diffusor transportiert wird. Der Diffusor ist bevorzugt an dem Behälter abgewandten Ende des insbesondere stiel- bzw. zylinderförmigen Dochtes angeordnet.

Der Diffusor ist wenigstens abschnittsweise mit einem Farbumschlagsmittel versehen, dass mit wenigstens einer Substanz der Duftstoffzubereitung derart zusammenwirkt, dass beim Kontakt der Substanz mit dem Farbumschlagsmittel eine Farbänderung auf und/oder im Diffusorabschnitt bewirkt wird.

Es ist bevorzugt, dass der Docht und/oder der Diffusor aus einem synthetischen Material und/oder einem Fasermaterial pflanzlicher Herkunft, insbesondere einem cellulose-basiertem Material, gebildet sind/ist. Es ist selbstverständlich möglich, dass der Docht und der Diffusor aus gleichen oder unterschiedlichen Materialien gefertigt sind.

Vorteilhaft ist es ferner, dass der Diffusor in der Aufsicht eine im Wesentlichen kreisrunde Grundfläche aufweist, so dass der Abstand zwischen Docht, der insbesondere in der Mitte der kreisrunden Grundfläche mit dem Diffusor verbunden ist, und äußerem Rand des Diffusors im Wesentlichen konstant ist. Hierdurch ergeben sich über die Grundfläche hinweg im Wesentlichen gleiche Diffussionsweglängen und -zeiten.

Um eine höhere Abgabe von Duftstoff zu erwirken, dann es vorteilhaft sein, dass das Verhältnis aus der mit Umgebungsluft in Kontakt stehenden Oberfläche des Diffusors und der mit der Umgebungsluft in Kontakt stehenden Oberfläche des Dochtes zwischen 1.000:1 und 1,25:1, bevorzugt zwischen 125:1 und 2,5:1 liegt. Der Diffusor ist aus einer Vielzahl von gleichartigen Lamellen gebildet, wobei ein Ende der Lamellen im bzw. am Docht fixiert ist. Es ist jedoch gemäß einer vorteilhaften Weiterentwicklung der Erfindung auch denkbar, dass der Diffusor aus wenigstens zwei Gruppen voneinander verschiedenen Lammellen gebildet ist.

Die Anzahl der Lamellen beträgt insbesondere mindestens 6, bevorzugt mindestens 50, insbesondere bevorzugt mindestens 100.

In einer weiteren besonders vorteilhaften Ausgestaltung der Erfindung weisen die Lammellen jeweils eine Oberfläche, bestehend aus Ober- und Unterseite einer Lammelle, von 0,5-100 cm², bevorzugt von 1-75 cm² insbesondere bevorzugt von 2-50 cm² auf. Die Lamellen sind an der Dochtasche schraubig (azyklisch) oder wirtelig (zyklisch) angeordnet. Bevorzugt beträgt der Winkel zwischen zwei benachbarten Lamellen zwischen 3° - 140°, bevorzugt zwischen 4° - 137,5°.

Der aus den Lammellen gebildete Diffusor kann in einer weiteren Ausgestaltung der Erfindung wenigstens eine, bevorzugt zwei, insbesondere bevorzugt drei Symmetrieebenen aufweisen.

Es ist besonders vorteilhaft, dass das Farbumschlagmittel ausgewählt ist aus der Gruppe der solvatochromen Farbstoffe

Es ist des Weiteren vorteilhaft, ein Farbumschlagsmittel auszuwählen, dass auf die Anwesenheit von insbesondere nicht toxischen Metallionen, vorzugsweise Ca, Mg, Al, Zn und/oder Fe, reagiert.

In einer weiteren, bevorzugten Ausführung der Erfindung ist das Farbumschlagmittel vor Kontakt mit der entsprechenden den Farbumschlag auslösenden Substanz, farblos.

Gemäß einer weiteren Ausgestaltung der Erfindung, ist es auch denkbar, dass der Docht und der Diffusor mit jeweils voneinander verschiedenen Farbumschlagsmitteln versehen sind. Eine erste Gruppe von Lamellen ist mit einem ersten Farbumschlagmittel und wenigstens eine zweite Gruppe von Lamellen ist mit einem zweiten Farbumschlagmittel versehen. Insbesondere ist es hierbei ebenfalls denkbar, dass das erste Farbumschlagmittel und das zweite Farbumschlagmittel bei den gleichen oder unterschiedlichen in der Duftstoffzubereitung vorhandenen Substanzen einen Farbumschlag zeigen.

Der Farbumschlagstoff kann durch die dem Fachmann aus dem Stand der Technik bekannten Verfahren, beispielsweise durch Sprühen oder Tauchen, auf den Docht und/oder den Diffusor aufgetragen werden.

| **Farbumschlagmittel (Docht/Diffusor)** | **Farbumschlag bewirkende Substanz in Duftstoffzubereitung** |
|---|---|
| Solvatochromer Farbstoff | Polares/ unpolares Lösungsmittel (Wasser/Ethanol) |
| Eriochromschwarz T (ERIO T) | Metallion |
| Murexid | Metallion |
| Metallphthalein, | Metallion |
| Brenzcatechinviolett | Metallion |
| 1-(2-Pyridylazo)-2-naphthol (PAN), | Metallion |
| 1-(2-Pyridylazo)-2-resorcin (PAR), | Metallion |
| Calconcarbonsäure (CC) | Metallion |
| Xylenorangel | Metallion |

Gemäß einer weiteren Ausführungsform der Erfindung, ist das Farbumschlagsmittel ERIO T, das mit Magnesiumionen einen roten Komplex bildet, wobei ERIO T ohne die Anwesenheit von Magnesiumionen blau ist. Tränkt man einen Docht und/oder Diffusor mit Mg(2+)-ERIO T so erscheinen der Docht und/oder der Diffusor blau. Enthält die Duftstoffzubereitung ein Komplexierungsmittel, welches Mg aus dem Farbstoffkomplex löst, bspw. EDTA oder Kronenether, so verfärbt sich der Docht und/oder der Diffusor rot, sobald die Duftstoffzubereitung mit dem Komplexbildner eindiffundiert. Selbstverständlich ist es auch möglich, dass ERIO T den Docht und/oder den Diffusor rot einfärbt und Mg2+ aus der Duftstoffzubereitung bei Kontakt den Docht und/oder den Diffusor blau verfärbt.

## Patentansprüche

1. Duftabgabesystem umfassend
- einen Docht
- mindestens einen Diffusor,
∘ wobei die mit Umgebungsluft in Kontakt stehende Oberfläche des Diffusors größer ist als die mit der Umgebungsluft in Kontakt stehenden Oberfläche des Dochtes und
- einen Behälter zur Aufnahme des Dochts
- eine Duftstoffzubereitung, die in dem Behälter bevorratet ist
- wobei die Duftstoffzubereitung in dem im Behälter aufgenommenen Zustand des Dochtes mittels Kapillarwirkung entgegen der Schwerkraftrichtung durch den Docht zum Diffusor transportiert wird,
**dadurch gekennzeichnet, dass**
wenigstens Abschnitte des Diffusors mit einem Farbumschlagsmittel versehen sind, dass mit wenigstens einer Substanz der Duftstoffzubereitung derart zusammenwirkt, dass beim Kontakt der Substanz mit dem Farbumschlagsmittel eine Farbänderung auf und/oder im Diffusorabschnitt bewirkt wird und dass der Diffusor aus einer Vielzahl von gleichartigen Lamellen gebildet ist, wobei ein Ende der Lamellen im bzw. am Docht fixiert ist, wobei die Lamellen an der Dochtachse schraubig oder wirtelig angeordnet sind, und wobei eine erste Gruppe von Lamellen mit einem ersten Farbumschlagmittel und wenigstens eine zweite Gruppe von Lammellen mit einem zweiten Farbumschlagmittel versehen sind.

2. Duftabgabesystem nach einem der vorherigen Ansprüche, **dadurch gekennzeichnet, dass** der Docht und/oder der Diffusor aus einem synthetischen Material und/oder einem Fasermaterial pflanzlicher Herkunft, insbesondere einem cellulose-basiertem Material, gebildet sind/ist.

3. Duftabgabesystem nach einem der vorherigen Ansprüche, **dadurch gekennzeichnet, dass** der Diffusor in der Aufsicht eine im Wesentlichen kreisrunde Grundfläche aufweist.

4. Duftabgabesystem nach einem der vorherigen Ansprüche, **dadurch gekennzeichnet, dass** das Verhältnis aus der mit Umgebungsluft in Kontakt stehenden Oberfläche des Diffusors und der mit der Umgebungsluft in Kontakt stehenden Oberfläche des Dochtes zwischen 1.000:1 und 1,25:1, bevorzugt zwischen 125:1 und 2,5:1 liegt.

5. Duftabgabesystem nach einem der vorherigen Ansprüche, **dadurch gekennzeichnet, dass** der Diffusor aus wenigstens zwei Gruppen voneinander verschiedenen Lammellen gebildet ist.

6. Duftabgabesystem nach einem der vorherigen Ansprüche, **dadurch gekennzeichnet, dass** die Anzahl der Lamellen mindestens 6, bevorzugt mindestens 50, insbesondere bevorzugt mindestens 100 beträgt.

7. Duftabgabesystem nach einem der vorherigen Ansprüche, **dadurch gekennzeichnet, dass** die Lammellen jeweils eine Oberfläche, bestehend aus Ober- und Unterseite einer Lammelle, von 0,5-100 cm², bevorzugt von 1-75 cm² insbesondere bevorzugt von 2-50 cm² aufweisen.

8. Duftabgabesystem nach einem der vorherigen Ansprüche, **dadurch gekennzeichnet, dass** der Winkel zwischen zwei benachbarten Lamellen zwischen 3° - 140°, bevorzugt zwischen 4°-137,5° liegt.

9. Duftabgabesystem nach einem der vorherigen Ansprüche, **dadurch gekennzeichnet, dass** der aus Lammellen gebildete Diffusor wenigstens eine, bevorzugt zwei, insbesondere bevorzugt drei Symmetrieebenen aufweist

10. Duftabgabesystem nach einem der vorherigen Ansprüche, **dadurch gekennzeichnet, dass** das Farbumschlagmittel ausgewählt ist aus der Gruppe der solvatochromen Farbstoffe.

11. Duftabgabesystem nach einem der vorherigen Ansprüche, **dadurch gekennzeichnet, dass** das Farbumschlagmittel vor Kontakt mit der entsprechenden den Farbumschlag auslösenden Substanz, farblos ist.

12. Duftabgabesystem nach einem der vorherigen Ansprüche, **dadurch gekennzeichnet, dass** der Docht und der Diffusor mit jeweils voneinander verschiedenen Farbumschlagsmitteln versehen sind.

13. Duftabgabesystem nach einem der vorherigen Ansprüche, **dadurch gekennzeichnet, dass** das erste Farbumschlagmittel und das zweite Farbumschlagmittel bei den gleichen oder unterschiedlichen in der Duftstoffzubereitung vorhandenen Substanzen einen Farbumschlag zeigen.

## Claims

1. A fragrance-releasing system comprising
- a wick
- at least one diffuser,
∘ the surface area of the diffuser which is in contact with the surrounding air being greater than the surface area of the wick which is in contact with the surrounding air, and
- a container for receiving the wick,
- a fragrance preparation which is stored in the container,
- the fragrance preparation being transported, when the wick is received in the container, opposite the direction of gravity through the wick to the diffuser by means of a capillary action,
**characterized in that**
at least portions of the diffuser are provided with a color-changing agent which interacts with at least one substance in the fragrance preparation such that when the substance is in contact with the color-changing agent, a color change is brought about on and/or in the diffuser portion, and **in that** the diffuser is formed of a plurality of identical lamellae, one end of the lamellae being fixed in or on the wick, the lamellae being arranged in a helical or whorled manner about the wick axis, and a first group of lamellae comprising a first color-changing agent and at least one second group of lamellae comprising a second color-changing agent being provided.

2. The fragrance-releasing system according to the preceding claim, **characterized in that** the wick and/or the diffuser is/are made of a synthetic material and/or a fiber material of plant origin, in particular a cellulose-based material.

3. The fragrance-releasing system according to one of the preceding claims, **characterized in that** the diffuser has, when viewed from the top, a substantially circular base.

4. The fragrance-releasing system according to one of the preceding claims, **characterized in that** the ratio of the surface area of the diffuser which is contact with the surrounding air and the surface area of the wick which is in contact with the surrounding air is between 1,000:1 and 1.25:1, preferably between 1.25:1 and 2.5:1.

5. The fragrance-releasing system according to one of the preceding claims, **characterized in that** the diffuser is formed of at least two groups of lamellae which are different from one another.

6. The fragrance-releasing system according to one of the preceding claims, **characterized in that** the number of lamellae is at least 6, preferably at least 50, particularly preferably at least 100.

7. The fragrance-releasing system according to one of the preceding claims, **characterized in that** the lamellae each have a surface area, consisting of an upper face and a lower face of a lamella, of 0.5-100 cm², preferably of 1-75 cm², particularly preferably of 2-50 cm².

8. The fragrance-releasing system according to one of the preceding claims, **characterized in that** the angle between two adjacent lamellae is between 3° and 140°, preferably between 4° and 137.5°.

9. The fragrance-releasing system according to one of the preceding claims, **characterized in that** the diffuser, formed of lamellae, has at least one, preferably two, particularly preferably three, planes of symmetry.

10. The fragrance-releasing system according to one of the preceding claims, **characterized in that** the color-changing agent is selected from the group of solvatochromic dyes.

11. The fragrance-releasing system according to one of the preceding claims, **characterized in that** the color-changing agent is colorless before contact with the corresponding substance that activates the color change.

12. The fragrance-releasing system according to one of the preceding claims, **characterized in that** the wick and the diffuser are each provided with color-changing agents which are different from one another.

13. The fragrance-releasing system according to one of the preceding claims, **characterized in that** the first color-changing agent and the second color-changing agent change color when the same or different substances are present in the fragrance preparation.

## Revendications

1. Système de libération de parfum comprenant :
- une mèche,
- au moins un diffuseur,
∘ la surface du diffuseur en contact avec l'air ambiant étant supérieure à la surface de la mèche en contact avec l'air ambiant, et
- un récipient destiné accueillir la mèche,
- une préparation de parfum stockée dans le récipient,
- la préparation de parfum dans la mèche, prélevée dans le récipient, étant transportée à travers la mèche jusqu'au diffuseur par capillarité contre le sens de la gravité,
**caractérisé en ce qu'**au moins des parties du diffuseur sont munies d'un produit à virage coloré, qui interagit avec au moins une substance de la préparation de parfum de sorte qu'au contact de la substance avec la préparation de parfum, il se produise un virage coloré sur et/ou dans la partie du diffuseur et **en ce que** le diffuseur soit constitué de plusieurs lamelles identiques, une extrémité des lamelles dans ou resp. sur la mèche étant fixée, les lamelles étant disposées en cercle ou en pas devis, et un premier groupe de lamelles étant muni d'un premier produit à virage coloré et au moins un deuxième groupe de lamelles étant muni d'un deuxième produit à virage coloré.

2. Système de libération de parfum selon une des revendications précédentes, **caractérisé en ce que** la mèche et/ou le diffuseur sont constitués d'un matériau synthétique et/ou d'un matériau fibreux d'origine végétale, en particulier d'un matériau à base de cellulose.

3. Système de libération de parfum selon une des revendications précédentes, **caractérisé en ce que** le diffuseur présente, vu du dessus, une surface essentiellement ronde.

4. Système de libération de parfum selon une des revendications précédentes, **caractérisé en ce que** le rapport de la surface du diffuseur en contact avec l'air ambiant avec la surface de la mèche en contact avec l'air ambiant se situe entre 1000:1 et 1,25:1, de préférence entre 125:1 et 2,5:1.

5. Système de libération de parfum selon une des revendications précédentes, **caractérisé en ce que** le diffuseur est constitué d'au moins deux groupes de lamelles différentes les unes des autres.

6. Système de libération de parfum selon une des revendications précédentes, **caractérisé en ce que** le nombre de lamelles est d'au moins 6, de préférence d'au moins 50, en particulier d'au moins 100.

7. Système de libération de parfum selon une des revendications précédentes, **caractérisé en ce que** les lamelles présentent respectivement une surface constituée d'une face supérieure ou d'une face inférieure, de 0,5 à 100 cm², de préférence de 1 à 75 cm², en particulier de 2 à 50 cm².

8. Système de libération de parfum selon une des revendications précédentes, **caractérisé en ce que** l'angle entre deux lamelles contiguës est entre 3° et 140°, de préférence entre 4° et 137,5°.

9. Système de libération de parfum selon une des revendications précédentes, **caractérisé en ce que** le diffuseur constitué de lamelles présente au moins un, de préférence deux, en particulier trois niveaux de symétrie,

10. Système de libération de parfum selon une des revendications précédentes, **caractérisé en ce que** le produit à virage coloré est choisi dans le groupe des colorants solvatochromes.

11. Système de libération de parfum selon une des revendications précédentes, **caractérisé en ce que** le produit à virage coloré est incolore avant contact avec la substance correspondante déclenchant le virage coloré.

12. Système de libération de parfum selon une des revendications précédentes, **caractérisé en ce que** la mèche et le diffuseur sont munis de produits à virage coloré différents l'un de l'autre,

13. Système de libération de parfum selon une des revendications précédentes, **caractérisé en ce que** le premier produit à virage coloré et le deuxième produit à virage coloré présentent un virage coloré en présence de substances présentes dans la préparation de parfum identiques ou différentes.
